# EUROPÄISCHE PATENTSCHRIFT

(11) **EP 1 596 708 B1**
(45) Veröffentlichungstag und Bekanntmachung des Hinweises auf die Patenterteilung: **12.10.2011**
(21) Anmeldenummer: 04706159.3
(22) Anmeldetag: 29.01.2004
(51) Int. Cl.: A61B 1/002

(54) **VERFAHREN ZUM MONTIEREN VON BAUELEMENTEN EINES OPTISCHEN SYSTEMS**
METHOD FOR ASSEMBLING COMPONENTS OF AN OPTICAL SYSTEM
PROCEDE POUR MONTER DES COMPOSANTS D'UN SYSTEME OPTIQUE

(30) Priorität: 18.02.2003 DE 10307904
(43) Veröffentlichungstag der Anmeldung: 23.11.2005
(73) Patentinhaber: Karl Storz GmbH & Co., 78532 Tuttlingen (DE)
(72) Erfinder: RUDISCHHAUSER, Jürgen, 78532 Tuttlingen (DE); RENNER, Klaus, 78576 Liptingen (DE); KUPFERSCHMID, Markus, 78576 Emmingen-Liptingen (DE)
(74) Vertreter: Weller, Wolfgang
(86) Internationale Anmeldenummer: PCT/EP2004/000765
(87) Internationale Veröffentlichungsnummer: WO 2004/073508

(56) Entgegenhaltungen:
- EP-A- 0 337 142
- DE-A- 3 738 451
- DE-A- 3 912 720
- DE-A- 19 732 991
- US-A1- 2002 007 111

## Beschreibung

Die Erfindung betrifft ein Verfahren zum Montieren von Bauelementen, insbesondere von Linsen, Abstandhaltern, Blenden, Filtern eines optischen Systems im Inneren eines Rohrschaftes eines Endoskops, wobei die Bauelemente von einem Stützelement aus geschrumpftem Material umgeben sind.

Ein derartiges Endoskop und ein solches Verfahren sind aus der DE 197 32 991 C2 bekannt.

Bei dem daraus bekannten Verfahren dient das schrumpffähige Material dazu, die Bauelemente des optischen Systems im Rohrschaft zu fixieren. Dazu werden die Bauelemente in ein diese teilweise umgebendes Stützelement aus schrumpffähigem Material gebracht und dieser Zusammenbau wird in den Rohrschaft eingeschoben. Dabei ist die Dimensionierung so, dass zwischen der Außenseite des Stützelements und der Innenseite des Rohrschafts ein kleiner Spalt verbleibt. Beim Schrumpfen des Materials expandiert dieses etwas radial, füllt den Spalt aus, so dass dadurch der Zusammenbau an der Innenseite des Rohrschafts fixiert ist.

Aus der DE 39 12 720 C2 ist ebenfalls die Verwendung eines Kunststoff-Schrumpfschlauches zur Positionierung der Elemente eines Relais-Linsensystems eines Endoskops bekannt. Das Material ist dabei so gewählt, dass es lichtundurchlässig, also undurchsichtig ist. Damit soll verhindert werden, dass Licht aus dem Lichtleiter in den Bereich des Relais-Linsensystems oder in den Bereich des Objektivs übertritt und dort zu Reflexionen bzw. Überstrahlungen führt. Zunächst können die Linsen des Relais-Linsensystems in einer richtigen Stellung positioniert werden. Anschließend wird der Schrumpfschlauch durch Anwendung von Wärme geschrumpft, so dass er die Linsen hält, ohne dass eine Linsenfassung im herkömmlichen Sinn erforderlich wäre.

Mit dieser Konstruktion soll es möglich sein, Endoskope äußerst kostengünstig herzustellen, daher ist beispielsweise auch vorgesehen, die Linsen aus Kunststoffmaterial herzustellen.

Bei der eingangs erwähnten DE 39 12 720 C2 ist Ziel, die an sich hochwertigen Bauelemente des optischen Systems unter Ausnützung der Schrumpfeigenschaften des diese Bauelemente umgebenden Materials an der Innenseite eines metallischen Rohrschafts zu fixieren.

Aufgabe der vorliegenden Erfindung ist ein Verfahren zum Montieren von Bauelementen dahingehend weiter zu optimieren, dass unter Einsatz schrumpfbarer Materialien eine Lagefixierung der optischen Bauelemente untereinander ermöglicht wird, die auch kontrollierbar ist.

Erfindungsgemäß wird die Aufgabe bei einem Verfahren durch die folgenden Schritte gelöst, nämlich Einbringen der Bauelemente in ein transparentes und schlauchförmiges schrumpffähiges Material unter Ausbilden eines Zusammenbaus, Schrumpfen des Materials zur Lagefixierung der Bauelemente untereinander, Überprüfen der Lage der Bauelemente untereinander durch das transparente geschrumpfte Material hindurch, und Einbringen des Zusammenbaus aus geschrumpftem schlauchförmigem Material mit den darin enthaltenen Bauelementen in den Rohrschaft.

Das optische System eines Endoskops ist durch eine Aneinanderreihung unterschiedlicher optischer Bauelemente aufgebaut. Eine besonders gute Abbildungsqualität kann man durch sogenannte Stablinsen erreichen. Dazu werden mehrere Stablinsen, die untereinander durch Abstandhalter getrennt sind, aneinandergereiht, wobei weitere Bauelemente wie Blenden, Filter oder abschließende Deckgläser oder Prismen zusätzlich vorgesehen sind.

Für eine gute Abbildungsqualität ist es nicht nur notwendig, diese Teile axial längs einer optischen Achse exakt zueinander auszurichten und zu fixieren, sondern es müssen auch deren relative Drehlagen zueinander unveränderbar sein. Im Laufe der Montage ist es sinnvoll, die optischen Abbildungsqualitäten eines solchen Linsensystems zu kontrollieren, um gegebenenfalls Systeme mit optischen Fehlausrichtungen zu eliminieren.

Üblicherweise wird die Qualitätsprüfung des optischen Systems erst nach kompletter Montage des Endoskops durchgeführt. Es ist dann sehr aufwendig, wenn optische Fehler auftreten, diese zu korrigieren, meist muss dazu das Endoskop komplett zerlegt werden.

Mit der vorliegenden Erfindung ist es nun möglich, einen Zusammenbau der optischen Bauelemente außerhalb des Endoskops herzustellen und diesen Zusammenbau visuell zu kontrollieren. Dazu wird ein transparentes schrumpffähiges Material eingesetzt, das in mehrfacher Hinsicht Vorteile gegenüber den aus dem Stand der Technik bekannten undurchsichtigen Materialien bietet. Zum einen kann schon bei dem Einbringen der einzelnen Bauelemente in das noch nicht geschrumpfte Material visuell die Lage der Bauelemente untereinander kontrolliert werden. Insbesondere kann erkannt werden, ob sich beispielsweise einzelne Filterelemente oder Blenden relativ verdreht haben, oder ob beispielsweise zwischen einem Abstandhalter und einer Stablinse axial noch ein Spalt vorhanden ist oder nicht.

Ferner kann die richtige Anordnung der Linsenbauteile, Linsen, Abstandshalter und gegebenenfalls Blenden, Filter und/oder Prismen, kontrolliert werden.

Nach dem Schrumpfen dieses Zusammenbaus ist erneut eine Kontrolle möglich, nämlich ob aufgrund des Schrumpfvorgangs irgendwelche Relatiweränderungen stattgefunden haben. Beim Schrumpfen bewegt sich das Material, das die optischen Elemente umgibt. Durch Vorsehen des transparenten Materials ist es nun erstmals möglich, auch nach dem Schrumpfen nochmals eine visuelle Kontrolle durchzuführen. Selbstverständlich sind auch schon Kontrollen in Richtung der optischen Achse, also durch die optischen Elemente hindurch, möglich. So kann schon bevor das optische System in dem Schaft montiert ist eine solche Vorkontrolle durchgeführt werden. Nach Einbringen des geschrumpften Zusammenbaus und endgültiger Positionierung dieses Zusammenbaus im Schaft kann dann nochmals eine Endkontrolle durchgeführt werden.

Dadurch wird die Montagesicherheit und auch die Montage als solche erleichtert und verbessert.

In einer weiteren Ausgestaltung werden sämtliche Bauelemente von einem einzigen Schlauch aus transparentem und geschrumpftem Material umgeben.

Diese Maßnahme hat den Vorteil, dass sämtliche Bauelemente in einen einzigen schlauchförmigen Körper eingebracht und dieser Zusammenbau nach dem Schrumpfen als Einheit gehandhabt werden kann, beispielsweise als Zusammenbau einfach in den Rohrschaft des Endoskops eingeschoben werden kann. Dieser Zusammenbau kann in den Endoskopschaft in die entsprechend zutreffende Drehposition eingebracht oder nach Einbringen in die richtige Drehlage gebracht werden. Bildet beispielsweise ein vorderer Abschluss ein Prisma mit seitlichem Blickwinkel, so kann die Lage, also seitlicher Blinkwinkel nach links, nach rechts, nach oben oder nach unten, entsprechend gewählt werden.

In einer weiteren Ausgestaltung werden die Bauelemente über das schlauchförmige geschrumpfte Material an der Innenseite des Rohrschafts fixiert.

Dazu sind nun zahlreiche Möglichkeiten gegeben, beispielsweise durch Klebemittelfixierung, wobei das Klebemittel vor Einbringen des Zusammenbaus aufgebracht werden kann, oder nach Einbringen durch radiale Bohrungen im Rohrschaft zur Fixierung eingebracht werden kann.

In einer weiteren Ausgestaltung werden die Bauelemente durch radiales Expandieren des geschrumpften Materials an der Innenseite des Rohrschafts fixiert.

Diese Maßnahme hat den Vorteil, dass der Effekt, wie er aus der DE 197 32 991 C2 bekannt ist, nun zusätzlich herangezogen wird, um den bereits "vorgeschrumpften" Zusammenbau durch einen weiteren Schrumpfvorgang an der Innenseite des Rohrschafts zu fixieren. Dabei findet im Wesentlichen eine weitere axiale Schrumpfung mit geringer radialer Expansion statt.

Das Maß des Schrumpfvorgangs ist durch die Art und die Dauer der Schrumpfbehandlung steuerbar. In einem ersten Vorschrumpfvorgang wird das Schrumpfphänomen dazu ausgenützt, um die in dem Schlauch eingebrachten Bauelemente untereinander zu fixieren. Nach Einschieben dieses Zusammenbaus in den Rohrschaft wird ein weiteres bzw. ein Nachschrumpfen durchgeführt, das lediglich dem Zweck dient, den Spalt zwischen der Außenseite des Zusammenbaus aus vorgeschrumpftem Schrumpfschlauch sowie den darin enthaltenen Bauelementen und der Innenseite des Rohrschafts in den dieser Zusammenbau eingeschoben ist, auszufüllen, um dadurch diesen Zusammenbau an dieser Innenseite des Rohrschafts zu fixieren, indem bei diesem Nachschrumpfen in axialer Richtung eine geringe Expansion in radialer Richtung auftritt. Hierfür können gewisse Vorbehandlungen des Schrumpfschlauchs vorgesehen sein, z.B. ein oder mehrere Wülste in der Form von im Querschnitt liegenden Ringen oder teilweisen Ringen. Diese geometrischen Abweichungen von der ansonstigen Zylinderform des Schrumgfschlauchs implizieren radiale Expansion der Geometrie des Schrumpfschlauchs bei seiner axialen Schrumpfung auch ohne Expansion des Materials als solches.

In einer Ausgestaltung des Verfahrens ist vorgesehen, den Zusammenbau aus Bauelementen und transparenten schrumpffähigem Material vor dem Schrumpfen in eine Haltevorrichtung einzulegen, in der der Zusammenbau ausgerichtet liegt.

Diese Maßnahme hat den Vorteil, dass durch die Haltevorrichtung zusätzliche Maßnahmen bereitgestellt werden, um den Zusammenbau ausgerichtet zu halten. Es ist auch möglich, den Zusammenbau nach Einlegen in der Haltevorrichtung nochmals auf korrekten Sitz zu überprüfen, bevor der Schrumpfvorgang ausgelöst wird.

Der in der Haltevorrichtung eingelegte Zusammenbau kann durch Anlegen eines Unterdruckes zusätzlich fixiert werden.

In einer weiteren Ausgestaltung des Verfahrens wird der Zusammenbau in eine Rinne der Haltevorrichtung eingelegt.

Dies ist besonders vorteilhaft, wenn lange Endoskopschäfte montiert werden sollen und insbesondere wenn die Gefahr besteht, dass aufgrund der Schwerkraft Durchbiegungen oder Ausbauchungen zu befürchten sind.

In einer weiteren Ausgestaltung wird der in die Haltevorrichtung eingelegte Zusammenbau durch Auflegen eines Gegenstandes beschwert.

Diese Maßnahme hat den Vorteil, dass nicht nur eine Abstützung in Schwerkraftrichtung durch Einlegen bewerkstelligt wird, sondern auch ein Verbiegen im Sinne eines Aufbäumens durch Auflegen des Gegenstands vor dem Schrumpfen verhindert werden kann.

In einer weiteren Ausgestaltung ist vorgesehen, dass der Gegenstand teilweise formschlüssig auf dem Zusammenbau aufgelegt wird.

Diese Maßnahme hat einen Vorteil, wenn sehr viele kleine Einzelteile zusammengelegt sind, die dazu neigen bei Bewegungen, beispielsweise beim Schrumpfen, ihre Position zu verändern.

Es versteht sich, dass die vorstehend genannten und die nachstehend noch zu erläuternden Maßnahmen nicht nur in der jeweils angegebenen Kombination, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert. Es zeigen:
- Fig. 1: einen Längsschnitt eines Zusammenbaus aus einem Schlauch aus transparentem und schrumpfförmigem Material und optischen Bauelementen, nämlich Stablinsen und Abstandhalter, vor dem Schrumpfen,
- Fig. 2: einen Querschnitt einer Haltevorrichtung, in der der in Fig. 1 dargestellte Zusammenbau eingelegt ist, und zwar während des Schrumpfens,
- Fig. 3: einen Längsschnitt durch ein Endoskop während der Montage in das gerade der in Fig. 1 dargestellte Zusammenbau, nachdem er in der in Fig. 2 dargestellten Haltevorrichtung 3 geschrumpft wurde, in den Rohrschaft eingebracht wird, und
- Fig. 4: stark vergrößert einen teilweisen Längsschnitt durch einen Schaft eines Endoskops in dessen Rohrschaft ein erfindungsgemäßer Zusammenbau eingeschoben ist, wobei auf der linken Hälfte ein Fixierung des Zusammenbaus an der Innenseite des Rohrschafts durch Klebestellen und auf der rechten Seite eine Fixierung durch ein weiteres Nachschrumpfen dargestellt ist.

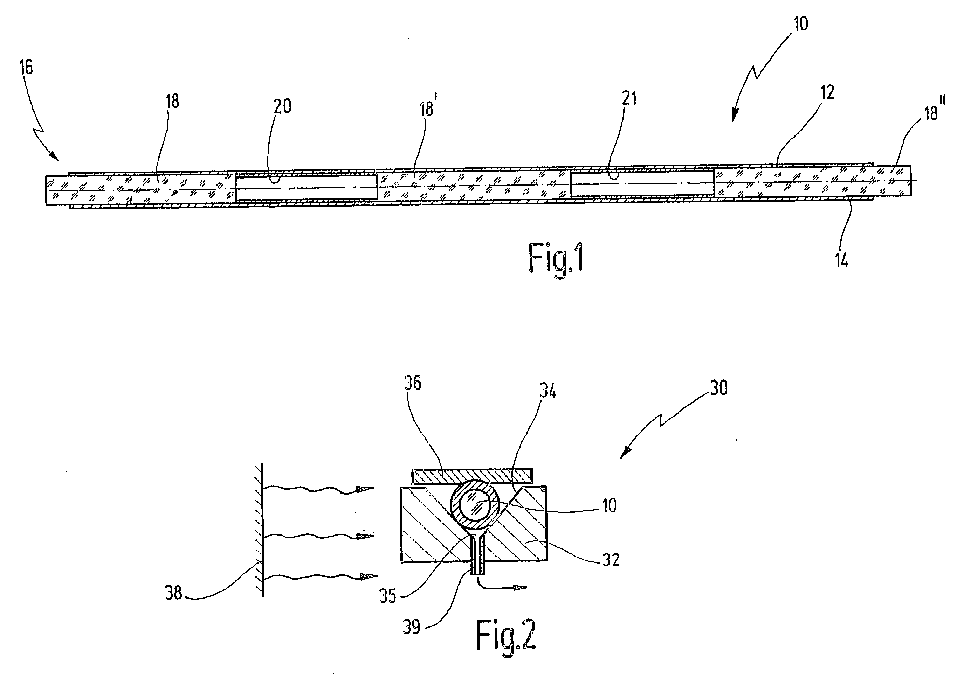

In Fig. 1 ist einer in seiner Gesamtheit mit der Bezugsziffer 10 bezeichneter Zusammenbau dargestellt, der einen Schlauch 12 aus transparentem und schrumpffähigem Material 14 aufweist. In den Schlauch 12 sind mehrere Bauelemente 16 eines optischen Systems eingeführt, und zwar, von links nach rechts gesehen, eine Stablinse 18, deren Außendurchmesser in etwa dem lichten Innendurchmesser des Schlauches 12 entspricht, ein rohrförmiger steifer Abstandhalter 20, eine weitere Stablinse 18', ein weiterer Abstandhalter 21 sowie eine weitere Stablinse 18".

Dieser Zusammenbau 10 ist nur beispielhaft, es können selbstverständlich weitere Bauelemente wie Filter, Blenden oder dergleichen zwischengelegt sein. Es ist auch möglich, endseitige Abschlussfenster oder, bei einem abgewinkelter Seitenblick, entsprechende Prismen vorzusehen.

Aufgrund der Transparenz des Materials 14 ist möglich, den gewünschten korrekten Sitz dieser Bauelemente 16 untereinander von der Außenseite her zu kontrollieren, beispielsweise ob die gegenüberliegenden Stirnseiten der beiden Stablinsen 18 und 18' exakt an dem Abstandhalter 20 anliegen.

Für den Schrumpfvorgang wird der Zusammenbau 10 in eine Haltevorrichtung 30 gelegt, wie sie in Fig. 2 dargestellt ist.

Die Haltevorrichtung 30 weist einen langerstreckten Körper 32 auf, dessen Länge zumindest der Länge des Zusammenbaus 10 entspricht.

Auf der Oberseite des Körpers 32 ist eine längserstreckende Rinne 34 ausgespart, die so ausgebildet ist, dass in diese Rinne der Zusammenbau 10 eingelegt werden kann, wobei der Zusammenbau 10 die Oberkante der Haltevorrichtung geringfügig überragt.

Auf diesen überragenden Bereich ist ein etwa plattenförmiger Gegenstand 36 aufgelegt, der zumindest teilweise formschlüssig auf der Oberseite des Zusammenbaus 10 aufliegt, diesen also quasi in die Rinne 34 drückt.

Dadurch ist der Zusammenbau 10 so in der Haltevorrichtung 30 eingelegt und fixiert, dass ein gleichmäßiges Schrumpfen des Materials 14 des Schlauchs 12 möglich ist, dennoch der Zusammenbau lagefixiert aufgenommen ist.

Zur Lagefixierung kann alternativ oder zusätzlich ein Unterdruck herangezogen werden. Dazu ist im Boden der Rinne 34 zumindest eine Öffnung 35 vorgesehen, die über einen Anschluss 39 mit einer hier nicht dargestellten Unterdruckquelle verbindbar ist.

Beim eigentlichen Schrumpfvorgang wird, wie das an sich bekannt ist, aus einer Energiequelle 38 Energie zugeführt, die veranlasst, dass das Material 14 des Schlauchs 12 schrumpft.

Eine Energiequelle ist beispielsweise Wärme, wenn das Material so ausgebildet ist, dass es bei Wärme schrumpft. Es ist natürlich auch möglich, die Haltevorrichtung 30 selbst zu erwärmen oder dies durch ein erwärmtes Fluid anzuströmen.

Nach dem Schrumpfen wird der Gegenstand 36 abgenommen und der nunmehr geschrumpfte Zusammenbau 10' aus der Haltevorrichtung 30 entnommen.

Aufgrund der Transparenz des Materials 14, die auch noch nach dem Schrumpfen vorhanden ist, ist es möglich, erneut von der Außenseite her den korrekten Sitz der einzelnen Bauelemente 16 untereinander erneut zu kontrollieren.

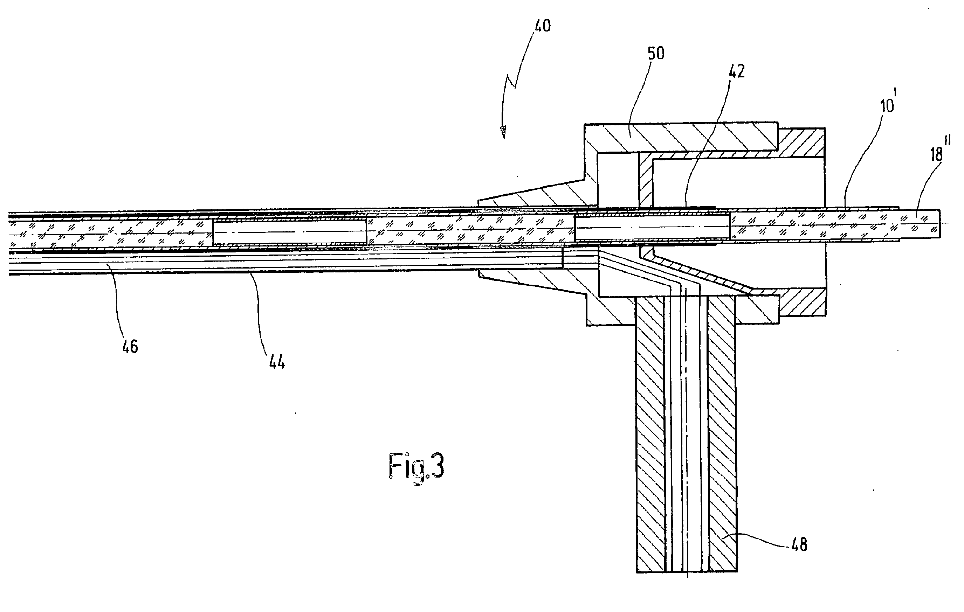

Anschließend wird der geschrumpfte Zusammenbau 10' in einen Rohrschaft 42 eines Endoskops 40 eingeschoben, wie das in Fig. 3 dargestellt ist.

Das in Fig. 3 dargestellte Endoskop 40 ist sehr schematisch dargestellt und weist neben dem Rohrschaft 42, das auch als Innenrohr bezeichnet wird, noch ein durchmessergrößeres Außenrohr 44 auf, das in einem Gehäuse 50 montiert ist. Der Rohrschaft 42 ist dabei im Inneren des Außenrohres 44 aufgenommen.

In einem etwa sichelförmigen Raum zwischen Rohrschaft 42 und Außenrohr 44 ist, wie das an sich ebenfalls üblich ist, ein Lichtleiter 46 angeordnet, der zu einem seitlich abgewinkelten Lichtleiteranschluss 48 führt. Im dargestellten Ausführungsbeispiel ist der Lichtleiter 46 aus einem Bündel an lichtleitenden Glasfasern ausgebildet. Der in Fig. 3 dargestellte Zustand ist ein Teilmontagezustand, an das rechte Ende wird dann noch die Okularmuschel angebracht, an das linke Ende gegebenenfalls Abschlusselemente oder dergleichen.

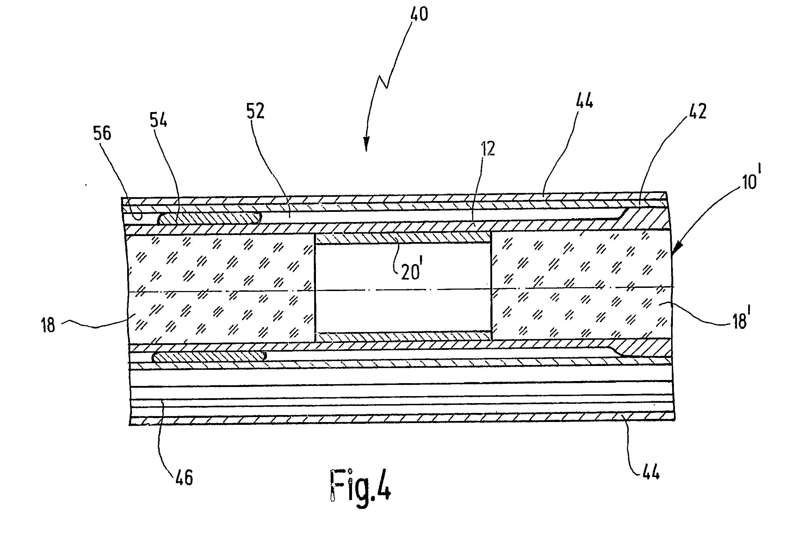

In Fig. 4 ist im stark vergrößerten Zustand ein Schnitt durch den Schaft eines Endoskops 40 dargestellt, wobei aus darstellerischen Gründen hier ein etwas kürzerer Abstandhalter 20' dargestellt ist, der die beiden Stablinsen 18 und 18' voneinander trennt.

Aus der Schnittdarstellung von Fig. 4 ist ersichtlich, dass in den Rohrschaft 42, der im Außenrohr 44 aufgenommen ist, der Zusammenbau 10' nach dem Schrumpfen eingeschoben ist. Dabei ist der Außendurchmesser so gewählt, dass zwischen der Außenseite des geschrumpften Schlauches 12 und der Innenseite 56 des Rohrschafts 42 ein geringer Spalt 52 vorhanden ist.

In Fig. 4 ist dieser Spalt 52 aus darstellerischen Gründen übermäßig groß dargestellt.

Die Spaltbreite ist so gewählt, dass der geschrumpfte Zusammenbau 10' einfach oder allenfalls unter geringem Widerstand in den Rohrschaft 42 eingeschoben werden kann.

In Fig. 4 ist auf der linken Seite dargestellt, dass der Zusammenbau 10' über ein Klebemittel 54 an der Innenseite 56 des Rohrschafts 42 fixiert ist. Das Klebemittel 54 kann entweder durch Öffnungen (hier nicht dargestellt), von der Außenseite her eingebracht werden oder vor Einschieben des geschrumpften Zusammenbaus 10' in den Rohrschaft 42 auf dieses aufgetragen werden.

Am rechten Ende von Fig. 4 ist dargestellt, dass der Zusammenbau 10' durch ein weiteres Schrumpfen des Schlauches und einer damit verbundenen radialen Expansion an der Innenseite 56 des Rohrschafts 52 fixiert ist, wobei der Schrumpfschlauch wie schon erwähnt geometrisch so gestaltet sein kann, z.B. durch Vorsehen von Wülsten, Einschnitten oder anderen Vorkehrungen, die ein Expandieren an vorbestimmten Stellen begünstigen, dass diese Expansion bei dem Nachschrumpfvorgang gezielt stattfindet.

Diese Möglichkeit wird dann gewählt, wenn es das Material 14 des Schlauchs 12 zulässt zwei Schrumpfvorgänge durchzuführen, nämlich ein erster oder "Vorschrumpfvorgang" zum Fixieren der Bauelemente untereinander, beispielsweise in der in Fig. 2 dargestellten Haltevorrichtung 30 und anschließend, nach Einschieben in den Rohrschaft 42, wie das in Fig. 4 dargestellt ist, durch ein weiteres Schrumpfen und radiales Expandieren zum Ausfüllen des Spalts 52.

## Patentansprüche

1. Verfahren zum Montieren von Bauelementen (16), insbesondere von Linsen (18, 18', 18''), Abstandhaltern (20, 20', 21), Blenden, Prismen, Filter eines optischen Systems im Inneren eines Rohrschafts (42) eines Endoskops (40), wobei die Bauelemente (16) von einem Stützelement aus geschrumpftem Material (14) umgeben sind, **gekennzeichnet durch** die Schritte:
- Einbringen der Bauelemente (16) in ein transparentes und schlauchförmiges schrumpffähiges Material (14) unter Ausbilden eines Zusammenbaus (10),
- Schrumpfen des Materials (14) zur Lagefixierung der Bauelemente (16) untereinander,
- Überprüfen der Lage der Bauelemente (16) untereinander **durch** das transparente geschrumpfte Material (14) hindurch, und
- Einbringen des Zusammenbaus (10') aus geschrumpftem schlauchförmigem Material (14) und den darin enthaltenen Bauelementen (16) in den Rohrschaft (42).

2. Verfahren nach Anspruch 1, **dadurch gekennzeichnet, dass** der Zusammenbau (10) aus Bauelementen und transparentem schrumpffähigem Material (14) vor dem Schrumpfen in eine Haltevorrichtung (30) eingelegt wird, in der der Zusammenbau (10) ausgerichtet liegt.

3. Verfahren nach Anspruch 2, **dadurch gekennzeichnet, dass** der in der Haltevorrichtung (30) eingelegte Zusammenbau (10) durch einen Unterdruck angezogen wird.

4. Verfahren nach Anspruch 2 oder 3, **dadurch gekennzeichnet, dass** der Zusammenbau (10) in eine Rinne (34) der Haltevorrichtung (30) eingelegt wird.

5. Verfahren nach einem der Ansprüche 2 bis 4, **dadurch gekennzeichnet, dass** der in die Haltevorrichtung (30) eingelegte Zusammenbau (10) durch Auflegen eines Gegenstands (36) beschwert wird.

6. Verfahren nach Anspruch 5, **dadurch gekennzeichnet, dass** der Gegenstand (36) teilweise formschlüssig auf den Zusammenbau aufgelegt wird.

7. Verfahren nach einem der Ansprüche 1 bis 6, **dadurch gekennzeichnet, dass** der Zusammenbau (10') nach Einbringen in den Rohrschaft (42) an dessen Innenseite (56) fixiert wird.

## Claims

1. Method for assembling components (16), in particular lenses (18, 18', 18"), spacers (20, 20', 21), diaphragms, prisms and filters of an optical system within a tubular shaft (22) of an endoscope (40), wherein said components (16) are surrounded by a support element of shrunk material (14), **characterized by** the steps of
- introducing the components (16) into a transparent and hose-like shrinkable material (14) to form an assembly (10),
- shrinking the material (14) for fixing a position of the components (14) relative to one another,
- checking the position of the components (16) relative to one another through the transparent shrunk material (14), and
- introducing the assembly (10') of shrunk hose-like material (14) and the components (16) contained therein into a tubular shaft (42).

2. Method of claim 1, **characterized in that** the assembly (10) of the components and the transparent shrinkable material (14) is, prior to shrinkage put into a support device (30), in which support device (30) the assembly (10) is aligned.

3. Method of claim 2, **characterized in that** the assembly (10) put in the support device (30) is attracted via an underpressure.

4. Method of claims 2 or 3, **characterized in that** the assembly (10) is put into a groove (34) of the support device (30).

5. Method of anyone of claims 2 to 4, **characterized in that** the assembly (10) put into the support device (30) is weighed down by posing an object (36) thereon.

6. Method of claim 5, **characterized in that** the object (36) is applied in a partially shape-closing manner onto the assembly.

7. Method of anyone of claims 1 through 6, **characterized in that** the assembly (10'), after inserting it into the tubular shaft (42) is fixed at an inside (56) of said tubular shaft (42).

## Revendications

1. Procédé pour monter des composants (16), en particulier des lentilles (18, 18', 18"), écarteurs (20, 20', 20"), obturateurs, prismes, filtres d'un système optique à l'intérieur d'un corps tubulaire (42) d'un endoscope (40), les composants (16) étant entourés par un élément de soutien en matériau (14) rétracté, **caractérisé par** les étapes suivantes :
- introduction des composants (16) dans un matériau (14) transparent, tubulaire et rétractable pour former un ensemble (10),
- rétraction du matériau (14) pour fixer en position les composants (16) entre eux,
- vérification de la position des composants (16) entre eux à travers le matériau (14) transparent rétracté, et
- introduction de l'ensemble (10') formé du matériau (14) tubulaire rétracté et des composants (16) qu'il contient dans le corps tubulaire (42).

2. Procédé selon la revendication 1, **caractérisé en ce que** l'ensemble (10) formé des composants et du matériau (14) transparent rétracté est placé, avant la rétraction, dans un dispositif de retenue (30) dans lequel l'ensemble (10) est aligné.

3. Procédé selon la revendication 2, **caractérisé en ce que** l'ensemble (10) placé dans le dispositif de retenue (30) est attiré par une dépression.

4. Procédé selon la revendication 2. ou 3, **caractérisé en ce que** l'ensemble (10) est placé dans une gouttière (34) du dispositif de retenue (30).

5. Procédé selon une des revendications 2 à 4, **caractérisé en ce que** l'ensemble (10) placé dans le dispositif de retenue (30) est alourdi par pose d'un objet (36).

6. Procédé selon la revendication 5, **caractérisé en ce que** l'objet (36) est posé sur l'ensemble en partie par complémentarité de forme.

7. Procédé selon une des revendications 1 à 6, **caractérisé en ce qu'**après introduction dans le corps tubulaire (42), l'ensemble (10') est fixé à son côté intérieur (56).
